Europäisches Patentamt

European Patent Office

Office européen des brevets

(19)

(11) Veröffentlichungsnummer: **0 086 997**
**B1**

(12) **EUROPÄISCHE PATENTSCHRIFT**

(45) Veröffentlichungstag der Patentschrift:
23.09.87

(21) Anmeldenummer: 83100850.3

(22) Anmeldetag: 29.01.83

(51) Int. Cl.⁴: **A 61 K 9/70**, A 61 L 15/03

(54) **Verfahren zur Herstellung einer pharmazeutischen Zubereitung in Form eines Polyacrylat-Films.**

(30) Priorität: 10.02.82 DE 3204551

(43) Veröffentlichungstag der Anmeldung:
31.08.83 Patentblatt 83/35

(45) Bekanntmachung des Hinweises auf die Patenterteilung:
23.09.87 Patentblatt 87/39

(84) Benannte Vertragsstaaten:
AT BE CH DE FR IT LI LU NL SE

(56) Entgegenhaltungen:
EP-A-0 020 905
DE-A-2 214 306
GB-A-1 296 241
GB-A-1 548 837

**Die Akte enthält technische Angaben, die nach dem Eingang der Anmeldung eingereicht wurden und die nicht in dieser Patentschrift enthalten sind.**

(73) Patentinhaber: BOEHRINGER INGELHEIM KG,
D-6507 Ingelheim am Rhein (DE)

(72) Erfinder: Zierenberg, Bernd, Dr., Veit-Stoss-Strasse 4, D-6507 Ingelheim (DE)

## Beschreibung

Die Erfindung betrifft ein neues Herstellungsverfahren für eine pharmazeutische Zubereitung in Form eines Polyacrylat-Films, die zur transdermalen Langzeitapplikation systemisch wirksamer pharmazeutischer Wirkstoffe geeignet ist.

Es ist bekannt, Arzneistoffe in Polymere einzubetten. So wird z. B. in der DE-A 2 006 696 ein Film beschrieben, der ganz allgemein aus Copolymerisaten von Acrylaten und Methacrylaten besteht und Wirkstoffe wie Antikontrazeptiva in homogener Vermischung enthält. Derartige Filme werden in Verbindung mit einem Pflaster appliziert, um die Wirkstoffe transdermal abzugeben.

Die US-A 4 076 798 betrifft eine pharmazeutische Zubereitung zur geregelten kontinuierlichen Applikation einer vorherbestimmten Wirkstoffmenge. Als Träger der pharmazeutischen Zubereitung wird ein biologisch abbaubares, hydrolisierbares Polyesterharz der Diglykolsäure vorgeschlagen.

Die DE-A 2 920 500 offenbart schließlich eine pharmazeutische Zusammensetzung in Form eines Polyacrylat-Films aus einem hautverträglichen, in Wasser quellbaren Copolymerisat aus Alkylesters der Acryl- und Methacrylsäure, worin der Wirkstoff in amorpher Form eingebettet ist und dessen charakteritisches Merkmal darin bestehen soll, daß er einen Wirkstoffkonzentrationsgradienten aufweist, wobei die Wirkstoffkonzentration im Polymer-Film mit zunehmender Entfernung von der Freigabefläche steigt. Die Herstellung dieser pharmazeutischen Zubereitung erfolgt in der Weise, daß man in der wässrigen Polyacrylat-Dispersion zunächst gegebenenfalls die Hilfsstoffe und - sofern es sich um gut wasserlösliche Arzneistoffe handelt - diese in der gewünschten Menge löst. Die Dispersion wird dann auf eine planare, begrenzte Fläche ausgegossen und evtl. bei erhöhter Temperatur zu einem Film trocknen gelassen. Anschließend werden die Wirkstoffe in Form einer organischen Lösung auf den Polyacrylat-Film aufgetragen und das Lösungsmittel verdampft. Erforderlichenfalls kann auf diesen Film, der nach entsprechender Konfektionierung die fertige pharmazeutische Zubereitungsform darstellt, zur Variation des Wirkstoffkonzentrationsprofils über den Filmquerschnitt auf der einen oder auf beiden Seiten des hergestellten Films erneut einmal oder mehrmals Dispersion aufgetragen und mit Wirkstoff beladen werden. Als besonders wichtige Faktoren, die die Freigabe-Geschwindigkeit beeinflussen, werden unter anderem die Bedingungen angesehen, unter denen der Polyacrylat-Film mit Wirkstoff beladen wird.

Neuere Untersuchungen haben nun ergeben, daß nach diesem 2-stufigen Verfahren der DE-A 2 920 500 zwar zunächst ein Polyacrylat-Film mit einem Wirkstoffkonzentrationsgradienten erhalten wird, daß aber insbesondere nach längerer Lagerung vor Gebrauch, durch innere Diffusionsvorgänge bedingt, ein Ausgleich des Konzentrationsprofils eintritt. Nach längerer Lagerung zeigen die Filme eine homogene Verteilung des Arzneistoffes im Polyacrylat-Film.

Im übrigen bringt bei einer kontinuierlichen Fertigung der zweite Behandlungsvorgang, nämlich die Beladung des Polyacrylat-Films mit einer Wirkstofflösung, technische Schwierigkeiten mit sich, da die Folie durch das Anlösen mit dem organischen Lösungsmittel erweicht und sich verwirft. Als Folge ergibt sich, daß die Stanzlinge aus einem derart hergestellten Film größere Standardabweichungen in der Freigaberate des Wirkstoffes aufweisen.

Um die genannten Schwierigkeiten bei einer schnellen kontinuierlichen Fertigung zu umgehen, wird die Filmherstellung erfindungsgemäß auf einen Schritt reduziert. Es wird ein Verfahren zur Herstellung einer pharmazeutischen Zubereitung in Form eines Polyacrylat-Filmes vorgeschlagen, das dadurch gekennzeichnet ist, daß ein gefriergetrockneter Latex aus einem Copolymerisat von Methyl- und/oder Äthylestern der Acryl- und Methacrylsäure mit einem durchschnittlichen Molekulargewicht von etwa 800 000 zusammen mit dem Arzneistoff in einem organischen Lösungsmittel oder Lösungsmittelgemisch aufgenommen, und anschließend zu einem Film ausgegossen und getrocknet wird.

Es wurde insbesondere gefunden, daß zur Herstellung eines transdermalen Freigabesystems auf der Basis eines Polyacrylat-Films es besonders darauf ankommt, ein in bestimmter Weise polymerisiertes Acrylat-Material zu verwenden. Das Acrylat-Copolymerisat muß durch Emulsionspolymerisation hergestellt sein. Es soll zweckmäßigerweise ein durshschnittliches Molekulargewicht von etwa 800 000 und eine Teilchengröße von ungefähr 140 nm haben. Auf andere Weise hergestellte Polyacrylate, z. B. lösungspolymerisierte oder blockpolymerisierte, sind für den erfindungsgemäßen Zweck nicht geeignet. Die Gewinnung des Feststoffes aus der Emulsion erfolgt am besten durch Gefriertrocknung. Hierbei bleiben die Polymerisatteilchen in ihrer Form und Größe erhalten.

Andere Formen der Polymerisatgewinnung aus dem Latex haben sich als weniger brauchbar herausgestellt. Ein geeignetes Ausgangsmaterial ist das auf dem Markt erhältliche Produkt Eudragit® E 30 D oder Plex® 47 91 D der Firma Röhm, Darmstadt. Natürlich können auch andere Polyacrylat-Latices verwendet werden, die bezüglich des Molekulargewichts und der Teilchengröße den vorbenannten Produkten entsprechen.

Der gefriergetrocknete Latex wird in einem organischen Lösungsmittel oder Lösungsmittelgemisch aufgenommen, das sowohl den Arzneistoff als auch das Polyacrylat zu lösen vermag. Als Lösungsmittel kommen niedere aliphatische Alkohole, Äther, Ketone, Ester, Kohlenwasserstoffe bzw. Halogenkohlenwasserstoffe in Frage, insbesondere solche, deren Siedepunkt unter 100°C liegen und die sich leicht verdampfen lassen. Auch Lösungsmittelgemische sind verwendbar. Durch Wahl eines geeigneten Lösungsmittels oder Lösungsmittelgemisches lassen sich die Viskositäten der Ausgangslösung verändern.

Das Freigabeverhalten der Arzneistoffe aus der filmförmigen Polymermatrix ist steuerbar, insbesondere wenn es sich um sauer oder basisch reagierende Arzneistoffe handelt. So kann man die Freigabegeschwindigkeit der Wirkstoffe durch Zugabe von saure oder basische Gruppen enthaltenden Polyacrylaten zu der Lösung, aus der der Film gegossen wird, variieren.

Die Filme sollen normalerweise eine Dicke von etwa 50 bis 200 µm besitzen. Die Temperatur, bei der die Trocknung der Lösung zu einem Film erfolgt, liegt normalerweise bei Raumtemperatur bis maximal zur Siedetemperatur des verwendeten Lösungsmittels oder Lösungsmittelgemisches, wobei man normalerweise versuchen wird, wegen der Zersetzlichkeit vieler Arzneiwirkstoffe und der Gefahr der Blasenbildung im Film die Trocknung bei möglichst tiefen Temperaturen vorzunehmen. Die Filmherstellung kann kontinuierlich oder diskontinuierlich erfolgen.

Die erhaltenen Filme werden in geeignete Stücke oder Stanzlinge zerschnitten und in üblicher Weise zur Herstellung von transdermalen Applikationsformen konfektioniert, etwa indem auf der einen Seite des wirkstoffhaltigen Films eine Stütz- und/oder Deckschicht und auf der anderen Seite eine Klebstoffschicht mit abziehbarem Schutzüberzug aufgebracht wird.

Die nach dem erfindungsgemäßen Verfahren hergestellten filmförmigen Zubereitungen wurden mit Filmen entsprechend der DE-A 29 20 500 in ihrem Freigabeverhalten verglichen. Es konnte festgestellt werden, daß keine wesentlichen Unterschiede zwischen beiden Filmtypen bestehen.

Die folgenden Beispiele erläutern die Erfindung in nicht beschränkender Weise.

Für die Beispiele wurde als Polyacrylat Eudragit® E 30 D der Firma Röhm und Haas, Darmstadt eingesetzt.

**Beispiel 1:** Wirkstoff Clonidin

a) Lösungsmittel Aceton
In einem geeignetan Gefäß werden 8 g gefriergetrocknetes Polyacrylat, 1 g Clonidin und 91 g Aceton gegeben und etwa 12 Stunden mit einem Magnetrührer gerührt, bis eine homogene, relativ viskose Lösung resultiert. Diese Lösung wird anschließend in Gießformen gegeben, wobei 190 mg/cm² aufgetragen werden. Bei Zimmertemperatur ist das Lösungsmittel Aceton nach etwa 6 Stunden verdampft. Man erhält einen glatten Film, der pro cm² 8,4 mg Polyacrylat und 1 mg Clonidin aufweist.

b) Lösungsmittel Acetessigester
Die Ausgangslösung setzt sich wie folgt zusammen: 6 g Polyacrylat, 0,53 g Clonidin und 93,5 g Acetessigester. Bei Auftragung von 390 mg/cm² führt dies zu einem Film mit 1 mg Clonidin und 11,6 mg Polyacrylat pro cm².

c) Lösungsmittel Methylenchlorid
Die Werte der Ausgangslösung: 3 g Polyacrylat, 0,37 g Clonidin und 96,6 g Methylenchlorid. Für eine Auftragungsmenge von 650 mg/cm² erhält man nach der Trocknung einen Film mit 1 mg Clonidin und 9,64 mg Polyacrylat pro cm².

Aus allen der drei untersuchten Lösungsmittel ergeben sich klare, transparente Filme, in denen der Arzneistoff molekular dispers vorliegt.

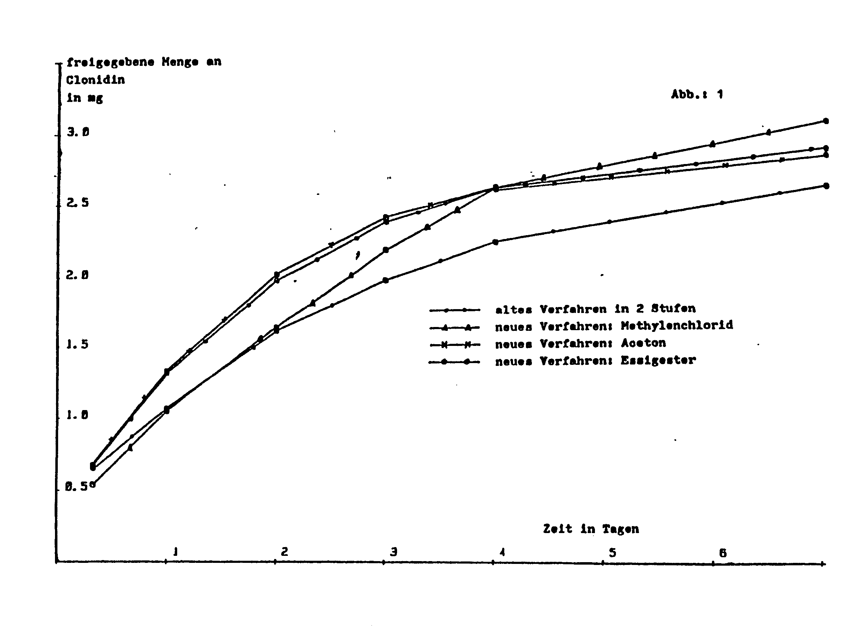

Die in vitro-Freigabemenge an Clonidin als Funktion der Zeit ist in Abb. 1 dargestellt; zum Vergleich ist die Freigabekurve für einen Film, der nach dem Zweistufenverfahren der DE-A 29 20 500 hergestellt wurde (Beladung mit dem Lösungsmittel Methanol) mit eingetragen.

Die nach dem neuen Verfahren hergestellten Filme zeichnen sich gegenüber den alten Filmen durch eine weit geringere Standardabweichung in der Freigaberate der einzelnen Stanzlinge untereinander aus. Die interindividuelle Schwankung ist 1 %, während sie früher bei etwa 6 % lag. Das heißt, die mit dem neuen Verfahren erhaltenen Filme weisen eine höhere Homogenität auf.

**Beispiel 2:** Wirkstoff Dihydroergotaminmethansulfonat

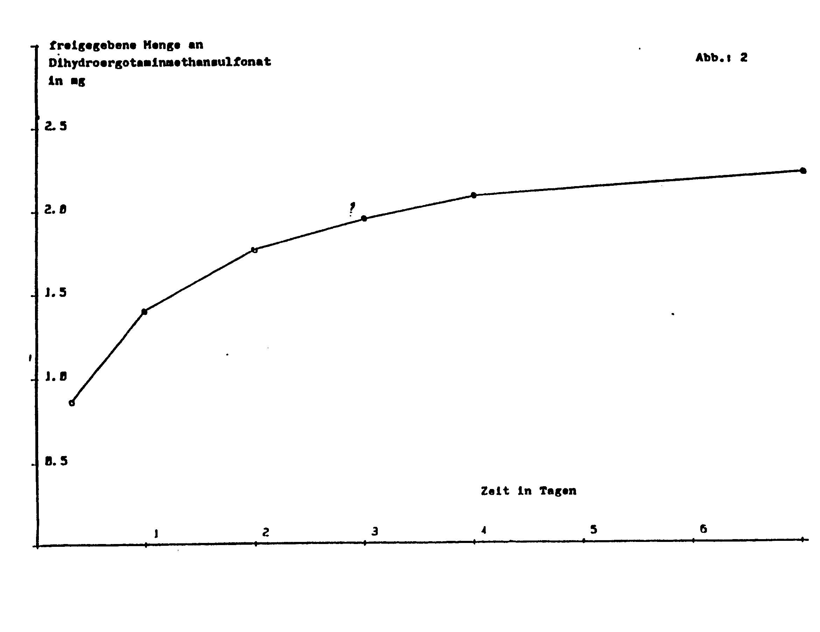

Lösungsmittel Methanol
Werte der Ausgangslösung: 8,75 g Polyacrylat, 1,375 g Dihydroergotaminmethansulfonat und 90,875 g Methanol. Für eine Auftragungsmenge von 244 mg/cm² erhält man nach der Trocknung einen Film mit 16,6 mg Polyacrylat und 0,96 g Dihydroergotaminmethansulfonat pro cm². Die in vitro freigegebene Menge an Wirkstoff als Funktion der Zeit ist in Abb. 2 dargestellt.

**Beispiel 3:** Wirkstoff Scopolamin

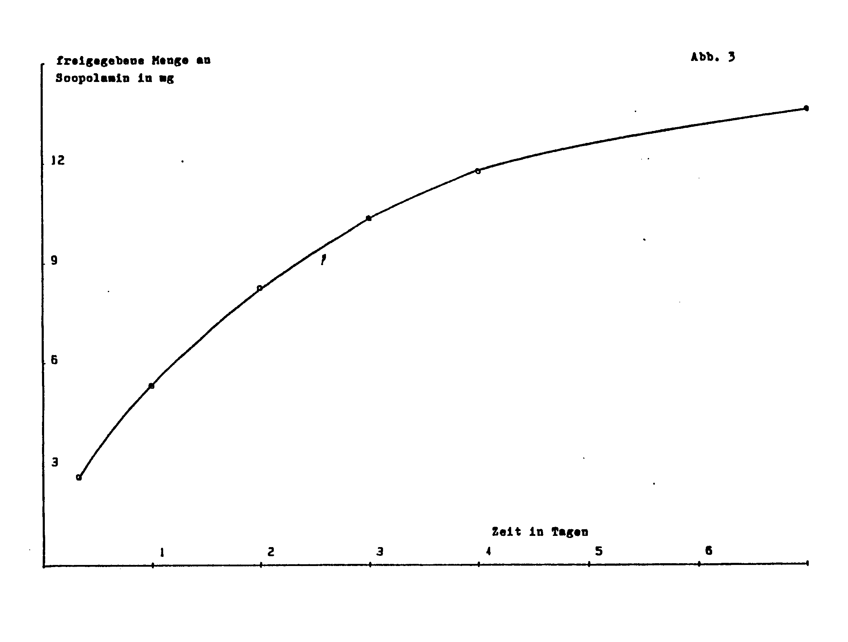

Lösungsmittel Aceton
Werte der Ausgangslösung: 12,0 g Polyacrylat, 3 g Scopolamin Base und 85 g Aceton. Für eine Auftragsmenge von 667 mg/cm² erhält man nach der Trocknung einen Film mit 22,3 mg Polyacrylat und 5,6 mg Scopolamin pro cm². Die in vitro-Freigabekurve ist in Abb. 3 wiedergegeben.

Im folgenden Beispiel wurde ein Gemisch aus Polyacrylaten verwendet. In dem Gemisch ist ein Teil des Produkts E 30 D durch ein Copolymerisat ersetzt, das aus 50 Mol-% Acrylsäure und 50 % Acrylsäuremethylester besteht und unter der Produktbezeichnung L 100 von der Firma Röhm und Haas, Darmstadt, vertrieben wird.

**Beispiel 4:** Wirkstoff Clonidin

a) Lösungsmittel Aceton/Äthanol (50 : 50 % w/w)

Die Ausgangslösung setzt sich wie folgt zusammen: 6,3 g Polyacrylat E 30 D, 0,7 g Polyacrylat L 100, 0,875 g Clonidin, 46 g Aceton und 46 g Äthanol. Bei Auftragung von 210 mg/cm² erhält man nach dem Trocknen einen Film mit 1 mg Clonidin und 7,85 mg Polyacrylat je cm².

b) Lösungsmittel Aceton/Äthanol (50 : 50 % w/w)

Die Werte der Ausgangslösung: 5,6 g Polyacrylat E 30 D, 1,4 g Polyacrylat L 100, 0,875 g Clonidin, 46 g Aceton und 46 g Äthanol. Bei Auftragung von 210 mg Gießlösung je cm² erhält man nach dem Trocknen einen Film von 1 mg Clonidin und 7,85 mg Polyacrylat je cm².

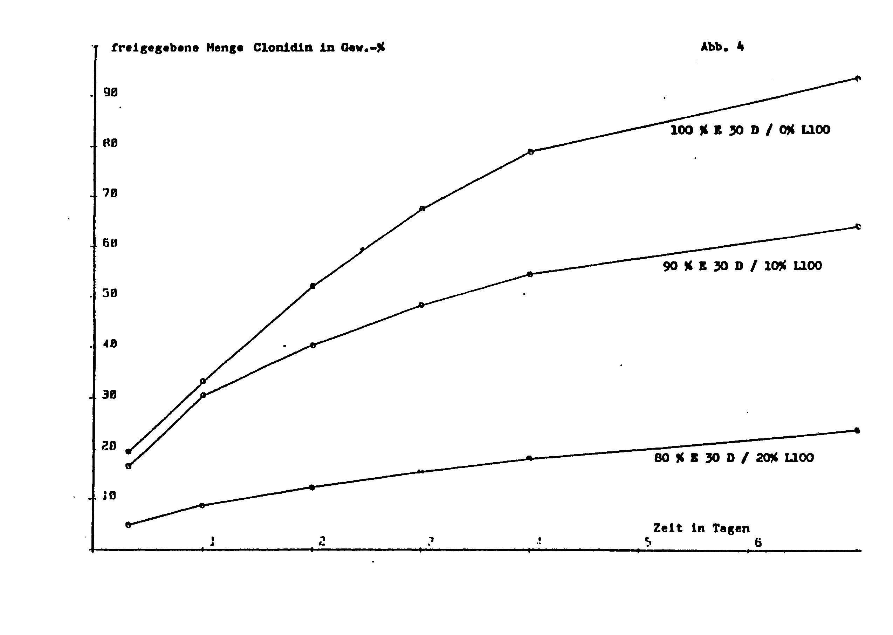

In der Abb. 4 sind die Freigabekurven der Beispiele 1a, 4a und 4b vergleichend dargestellt. Man erkennt, daß die Diffusion durch Salzbildung des Clonidin an die Polymere Acrylsäure, eingebettet in das Polyacrylat E 30 D, stark verlangsamt wird. Als ungefähres Maß läßt sich angeben, daß bei doppelter äquimolarer Menge an Carboxylgruppen im Polymeren, bezogen auf die Menge an Wirkstoffbase, die Freigabegeschwindigkeit auf etwa 1/5 abfällt.

**Patentansprüche**

1. Verfahren zur Herstellung einer pharmazeutischen Zubereitung in Form eines Polyacrylatfilms, dadurch gekennzeichnet, daß ein gefriergetrockneter Latex aus einem Copolymerisat von Methyl- und/oder Äthylestern der Acryl- und Methacrylsäure mit einem durchschnittlichen Molekulargewicht von etwa 800 000 zusammen mit dem Arzneistoff in einem organischen Lösungsmittel oder Lösungsmittelgemisch aufgenommen, in eine homogene Lösung überführt und anschließend zu einem Film ausgegossen und getrocknet wird.

2. Verfahren nach Anspruch 1, dadurch gekennzeichnet, daß die Filmherstellung kontinuierlich oder diskontinuierlich erfolgt.

3. Verfahren nach einem der vorhergehenden Ansprüche, dadurch gekennzeichnet, daß die Trocknung des Films bei Temperaturen von Raumtemperatur bis maximal zur Siedetemperatur des Lösungsmittels oder Lösungsmittelgemisches erfolgt.

4. Verfahren nach einem der vorhergehenden Anspüche, dadurch gekennzeichnet, daß der Filmgießlösung saure oder basische Gruppen enthaltende Polyacrylate zugesetzt werden.

**Claims**

1. Process for the production of a pharmaceutical preparation in the form of a polyacrylate film, characterised in that a freeze-dried latex consisting of a copolymer of methyl and/or ethyl esters of acrylic and methacrylic acid with an average molecular weight of about 800,000 is taken up, together with the pharmaceutical substance, in an organic solvent or mixture of solvents, then converted into a homogeneous solution and subsequently poured out and dried to form a film.

2. Process as claimed in claim 1, characterised in that the production of the film is carried out continuously or discontinuously.

3. Process as claimed in one of the preceding claims, characterised in that the drying of the film is effected at temperatures ranging from ambient temperature to, at most, the boiling temperature of the solvent or mixture of solvents.

4. Process as claimed in one of the preceding claims, characterised in that polyacrylates containing acidic or basic groups are added to the pouring solution for the film.

**Revendications**

1. Procédé pour la préparation d'une formulation pharmaceutique sous forme d'une pellicule ou film de polyacrylate, caractérisé en ce qu'un latex lyophilisé obtenu à partir d'un copolymérisat d'esters méthylique et/ou éthylique des acides acrylique et méthacrylique présentant un poids moléculaire moyen d'environ 800.000 est repris conjointement avec la substance médicinale dans un solvant ou dans un mélange de solvants organiques, transformé en une solution homogène et coulé ensuite pour former un film, puis séché.

2. Procédé selon la revendication 1, caractérisé en ce que la préparation de la pellicule s'effectue en continu ou en discontinu.

3. Procédé selon l'une des revendications précédentes, caractérisé en ce que le séchage de la pellicule s'effectue à des températures allant de la température ambiante jusqu'au maximum à la température d'ébullition du solvant ou du mélange de solvants.

4. Procédé selon l'une des revendications précédentes, caractérisé en ce que l'on ajoute à la solution de coulée de la pellicule des polyacrylates contenant des groupes acides ou basiques.

freigegebene Menge an
Clonidin
in mg

Abb.: 1

3.0

2.5

2.0

1.5

1.0

0.5

———•——— altes Verfahren in 2 Stufen
—▲—▲— neues Verfahren: Methylenchlorid
—×—×— neues Verfahren: Aceton
—●—●— neues Verfahren: Essigester

Zeit in Tagen

1    2    3    4    5    6

0 086 997

freigegebene Menge an
Dihydroergotaminmethansulfonat
in mg

Abb.: 2

Zeit in Tagen

freigegebene Menge an
Soopolamin in mg

Abb. 3

Zeit in Tagen

0 086 997

freigegebene Menge Clonidin in Gew.-%

Abb. 4

100 % E 30 D / 0% L100

90 % E 30 D / 10% L100

80 % E 30 D / 20% L100

Zeit in Tagen